# EUROPEAN PATENT SPECIFICATION

(11) **EP 1 477 173 B1**
(45) Date of publication and mention of the grant of the patent: **19.12.2007**
(21) Application number: 04011588.3
(22) Date of filing: 14.05.2004
(51) Int. Cl.: A61K 31/7024, A23L 1/304, C07H 11/04

(54) **Use of monosaccharide posphates for improving the intestinal function**
Verwendung von Monosachharidphosphaten zur Verbesserung der intestinalen Funktion
Utilisation des phosphates de monosaccharides pour ameliorer la fonction intestinale

(30) Priority: 11.11.2003 JP 2003380652; 11.11.2003 JP 2003380651; 16.05.2003 JP 2003138985; 16.05.2003 JP 2003138987
(43) Date of publication of application: 17.11.2004
(73) Proprietor: KAO CORPORATION, Tokyo 103-8210 (JP)
(72) Inventor: Fujinaka, Hidetake, Haga-gun Tochigi 321-3497 (JP); Nakamura, Junji, Haga-gun Tochigi 321-3497 (JP); Murase, Daiki, Haga-gun Tochigi 321-3497 (JP); Souno, Hatsumi, Haga-gun Tochigi 321-3497 (JP); Kobayashi, Hisataka, Haga-gun Tochigi 321-3497 (JP)
(74) Representative: HOFFMANN EITLE

(56) References cited:
- EP-A- 1 477 171
- PATENT ABSTRACTS OF JAPAN vol. 0145, no. 71 (C-0790), 19 December 1990 (1990-12-19) & JP 2 249468 A (KAO CORP), 5 October 1990 (1990-10-05)
- PATENT ABSTRACTS OF JAPAN vol. 2003, no. 01, 14 January 2003 (2003-01-14) & JP 2002 253170 A (EZAKI GLICO CO LTD; OJI CORNSTARCH CO LTD), 10 September 2002 (2002-09-10)
- PATENT ABSTRACTS OF JAPAN vol. 1996, no. 07, 31 July 1996 (1996-07-31) & JP 8 056614 A (UNITIKA LTD), 5 March 1996 (1996-03-05)

## Description

### Field of the Invention

The present invention relates to an alternative agent with vitamin D-like activity and an improving agent for intestinal function.

### Background of the Invention

It has been known that a vitamin D group includes, in addition to vitamin D itself, vitamin D matabolites (such as 1α,25-dihydroxyvitamin D (active form), and 1α,24-dihydroxyvitamin D). At present, 1α,25-dihydroxyvitamin D is under investigation for wide use as therapeutic drug for treatment of skin disease and other diseases such as parathyroid disorder, malignant tumors and bone diseases. In research field of skin disorder, for example, it has been reported that 1α,25-dihydroxyvitamin D is quite effective for treatment of psoriasis vulgaris (J. Kobayashi, et al., MB Derma. 2:53-62, 1997). Also, in particular, 1 α,25-dihydroxyvitamin D is considered to have effectiveness for treatment of malignant tumors such as prostate cancer, colon cancer, breast cancer and blood cancer (T. Akizawa, et al., Vitamin D Update 2001, Mediculture, 2001). Based on epidemiological investigations, it has also been reported that deficiency of 1α,25-dihydroxyvitamin D could affect emergence and development of these diseases, and although there is difference in effectiveness depending on malignant tumor type, the substantial efficacy was obtained by administration of 1α,25-dihydroxyvitamin D (Cummingham, D. et al., Br. Med. J., 291:1153-1155(1985)).

Also, it has long been known that dietary deficiency of vitamin D develops rachitis in accordance with decrease in mineral concentration in blood, and 1α,25-dihydroxyvitamin D has also been used as therapeutic drug for bone metabolic disease.

There are also publications describing that 1α,25-dihydroxyvitamin D -enriched composition can be prepared by mixing mushrooms containing high level of vitamin D or vitamin D itself with homogenate of animal liver and kidney (JP-A-10-236971, JP-A-11-158074). Those procedures intend to convert biologically inactive intact vitamin D by the addition of vitamin D activating enzymes contained in liver and kidney to synthesize 1α,25-dihydroxyvitamin D. However, the above method for providing an effectively 1α,25-dihydroxyvitamin D-enriched preparation has some problems, such as the above organs also contain 24-hydroxylase which denaturate the 1α,25-dihydroxyvitamin D, and the tissue homogenates, containing many kinds of substances, are those prepared only by the addition of substrate vitamin D and incubation.

On the other hand, it has been known that repetitive dosing of 1α,25-dihydroxyvitamin D may cause some adverse reactions, for example, loss of body weight due to getting hypercalcemia. Therefore, further caution is required in treatment with 1α,25-dihydroxyvitamin D (Weber K. et al: J. Bone Miner. Res., 10(4). 639-651(2001)).

On the other hand, Protein-Energy- Malnutrition (PEM) has been observed in 30 to 40 % of the elderly people with declined daily living activities, and as one of the reasons, depression of digestion/absorption activity of intestine is pointed out (Report: The Elderly Health Care Business Promotion Program "Study on nutrition control service for the elderly", supported by the Ministry of Health, Labor and Welfare in Japan). It was revealed that extended period of post operational fasting or PEM of the elderly patients caused morphological change in gastrointestinal mucosa, depression of barrier function, depression of gastrointestinal immune response and loss of balanced bacterial flora in intestine, resulting in tendency for endotoxin and bacteria to pass through intestinal wall (N. Hosoya, et al.: Digestion/Absorption, Daiichi Publishing, 2002), and risk of causing serious disease is believed to increase. Also, there is a report that depression of digestion/absorption activity of intestine by aging was a trigger of bone metabolic disease of elderly people (K. Uenishi, et al.: Abstracts of General Conference of The Japanese Society of Nutritional Food, 54, 109, 2000).

As to furnish nutrition for the elderly, infants and invalids, it has been reported that a digestion/absorption promoting agent, consisting of phosphatidylchorine as an active ingredient, is effective to increase both body weight and mass of small intestine of the individual (JP-A-2002-167331), and also, a nutritional composition, consisting of lactoperoxydase and lactoferrin and acting as suppressive to lactase and enhancive to both sucrase and maltase, has efficacy to maturation and restoration of intestinal functions (JP-A-2001-29011). However, as these components are liable to be denatured and are contained in the particular foods with only limited amount, close attention should be paid to storage stability in the compounded products and on the product manufacturing process, moreover, especially due to versatile physiological activities of lactoferrin, careful attention should be paid to safety and adverse reaction thereof. Thus, not negligible number of problems is still remained.

On the other hand, as to physiological functions of a sugar-phosphate esters, the following articles are reported; pH buffering action (JP-A-62-243675), application to infusion fluids (JP-A-02-250821), utilization as calcium fortifiers (JP-A-2002-145893, JP-A-2002-253170), application to preservatives of red blood cell (JP-A-62-158221), a therapeutic agent containing hexose calcium phosphate as an active ingredient for hypocalcemia and bone metabolic disorders (JP-A-64-22), an intestinal absorption promoter composed of inorganic substances containing a sugar-phosphate esters (JP-A-2-249468), a mineral taste improver consisting of a sugar-phosphate esters or salts thereof (JP-A-2003-79337). However, in these reports, there is no detailed description whether sugar-phosphate esters have vitamin D-like activity, nor how a sugar-phosphate esters work for age-related depression of intestinal function.
JP 8 056614 A reveals a food or beverage containing fructose-1,6-diphoshonic acid di-magnesium salt which enhances the absorption of magnesium.

### Summary of the Invention

The present invention provides the use of a monosaccharide phosphate ester or a salt thereof for the manufacture of an agent for providing vitamin D-like activity for preventing or curing skin disease, parathyroid disease, malignant tumor and bone disease.

Furthermore, the present invention provides the use of a monosaccharide phosphate ester or a salt thereof for the manufacture of an agent for the improvement of age-related depression of intestinal function.

### Brief Description of the Drawings

Fig.1 shows effects of glucose 1-phosphate sodium salt and 1α,25-dihydroxyvitamin D on expression of CYP3A gene.
Fig.2 shows effects of glucose 1-phosphate sodium salt and 1α,25-dihydroxyvitamin D on expression of 24-hydroxylase gene.
Fig.3 shows effects of glucose 1-phosphate sodium salt and 1α,25-dihydroxyvitamin D on expression of CaT1 gene.
Fig.4 shows effects of glucose 1-phosphate sodium salt on expression of intestinal calbindin D9K in aged rats.

### Detailed Description of the Invention

The present invention provides therapeutic drugs and foods which have both activity similar to 1α,25-dihydroxyvitamin D and safety for long term administration without adverse reaction, and also provides the same that can restore or improve age-related depression of digestion and absorption ability of intestinal canal.

The present inventors have performed screening study for 1α,25-dihydroxyvitamin D activity by means of detecting expression products as indicator of targeting gene of 1α,25-dihydroxyvitamin D. More specifically, 1α,25-dihydroxyvitamin D binds to a vitamin D receptor (VDR) in a target cell, and further, binds to a retinoid X receptor to form a heterodimer. Then, binding of this complex with vitamin D responding sequence on a gene, activates transcription of genes, having this vitamin D responding sequence as a promoter, to express various physiological actions(Y. Ohyama et al.: J. Biol. Chem., 269, 10545-10550, 1994). The following substances are known as targeting genes of said 1α,25-dihydroxyvitamin D; 24-hydroxylase, CYP3A, CaT1, calbindin D9K, calbindin D28K, osteocalcin, osteopontin and the like. Substances which enhance expression of said targeting genes were screened using various compounds, and surprisingly, it was found that a monosaccharide phosphate ester or a salt thereof had excellent activity to enhance expression of targeting genes, and was also found usefulness as alternative agents with vitamin D-like activity.

Moreover, the present inventors have noticed calbindin D9K, which is a protein having calcium-binding domain, and expresses in intestinal tissue with relatively high level, and known to decline its expression level in accordance with aging (H. J. Armbrecht et al: Am. J. Physiol., 277, g41-g47, 1999). Then, an intestinal function promoter was searched, using aged rats, by measuring decreased level of calbindin D9K as the indicator, to find out that a monosaccharide phosphate ester or a salt thereof significantly recover age-related depressing of calbindin D9K expression, and useful as intestinal function promoters.

Monosaccharide phosphate esters used for an alternative agent with vitamin D-like activity and an improving agent for intestinal function of the present invention include more specifically, hexose phosphate ester or pentose phosphate ester. Said monosaccharide phosphate ester includes glucose-1-phosphate, glucose-6-phosphate, glucose-1,6-diphosphate, maltose-1-phosphate, fructose-1-phosphate, fructose-6-phosphate, galactose-1-phosphate, galactose-6-phosphate, lactose-1-phosphate and sucrose-1-phosphate. Among them, glucose-1-phosphate, glucose-6-phosphate, and fructose-6-phosphate are more preferable.

Among these monosaccharide phosphate esters, glucose-1-phosphate is an intermediate product in glycolytic pathway from glycogen to TCA cycle in sugar metabolism, and is contained in many ordinary consumed foodstuff (Fisheries Science, 64(1), 125-130, 1998, Anal. Biochem, 176(2), 449-456 1989). Further, there have been many reports on very high-safety of glucose-1-phosphate by toxicity study of single and repeated administration (Fournier E. et al: Therapie, 26(1):27-37, 1971, and so on). Thus, glucose-1-phosphate can be consumed safely in both medical field and food industry, and is most preferable.

Salts of these monosaccharide phosphate esters include a sodium salt, a potassium salt, a calcium salt, an iron salt, a magnesium salt. Among them, alkaline metal salts such as a sodium salt and a potassium salt are preferable because of high solubility in water, and a sodium salt is more preferable. These monosaccharide phosphate esters or salts thereof may be in hydrated form.

These monosaccharide phosphate esters can be manufactured in large scale at low cost by means of an immobilized enzyme method or a fermentation method using microbiology, as described, for example, in JP-A-61-22096, JP-A-1-639589 and JP-A-2002-300899. As an improving agent for intestinal function of the present invention, a monosaccharide phosphate ester or a salt thereof, produced and purified by the above method, can be used and further the unprocessed fermentation product can also be used as it is.

As described in the later Examples, a monosaccharide phosphate ester or a salt thereof promotes expression of 24-hydroxylase, CYP3A and CaT1 genes, which are target genes of 1α,25-dihydroxyvitamin D, and presents vitamin D-like activity. As a consequence, a monosaccharide phosphate ester or a salt thereof is quite valuable as an alternative agent with vitamin D-like activity, and also can be used for therapeutic drug and foods which require vitamin D-like activity, i. e. preventive or curative medicines and foods for patients with skin disease, parathyroid disease, malignant tumor and bone disease.

And also, a monosaccharide phosphate ester, especially glucose-phosphate ester or a salt thereof, as will be described in the following Examples, has activity to revive age-related depression of calbindin D9K expression in intestine. On the other hand, as described above, calbindin D9K is a protein known to decline expression by aging. In consequence, a monosaccharide phosphate ester or a salt thereof is useful as an calbindin D9K fortifier, and also as therapeutic drugs or foods to be used as an improving agent of intestinal function, for example intestinal absorption function depressed by aging.

A type of formulation of a vitamin D-like active agent and an improving agent for intestinal function of the present invention may be the same as general formulation for oral administration, and preferable one includes, flour, tablet, granule, powder, capsules, water solution and suspension.

A vitamin D-like active agent or an improving agent for intestinal function of the present invention can be used as functional foods, without any specific requirement of product type, including fruit juice; vegetable juice; seasonings such as source, soybean paste and soy source; soybean foods such as soy milk and fermented soybean; emulsified foods such as cream, dressing, mayonnaise and margarine; processed aquatic foods; processed meat products; beverages such as refreshing drink, alcoholic liquors, coffee, hot chocolate, black tea, green tea, fermented tea, half-fermented tea, powder drinks and functional drinks; pickles; noodles; soups including powdered soup; jerry; dairy products such as cheese, yoghurt and milk; wheat flour foods such as bread and cake; confections such as snacks, chewing gum, candy and chocolate; health foods such as tablets and granules.

An agent with vitamin D-like activity of the present invention can be blended with, for example, vitamins such as tocopherol, ascorbic acid and derivatives thereof, phylloquinone, menaquinone, menadione and its derivatives, *β*-carotene, vitamin A, thiamine, riboflavin, vitamin B6, cyanocobalamin and folic acid; sugars such as oligosaccharide, hexose, pentose and sugar alcohol; alcohols such as ethanol; organic acids such as citric acid and malic acid; proteins and peptides such as milk whey protein, milk-derived protein like casein and soybean protein; flavonoides such as catechin and isoflavone; proteoglycans such as chondroitin sulfate and gentamycin sulfate; extracts or galenicals of such as clove, juniper berry and arborvitae. Also, combined use is possible with, for example, a thickening agent, a gelatinizing agent, carrageenan as an emulsion stabilizer, carboxymethylcellulose, guar gum, pectin, xanthan gum, Arabic gum, locust bean gum, tragacanth gum, gelin gum, alginic acid, dextran, pullulan and curdlan.

An improving agent of the present invention for intestinal function can be blended with, for example, vitamins such as vitamin D, 1α,25-dihydroxyvitamin D, 25-hydroxyvitamin D, tocopherol, ascorbic acid and derivatives thereof, phylloquinone, menaquinone, menadione and its derivatives, β-carotene, vitamin A, thiamine, riboflavin, vitamin B6, cyanocobalamin and folic acid; sugars such as oligosaccharide, hexose, pentose and sugar alcohol; alcohols such as ethanol; organic acids such as citric acid and malic acid; proteins and peptides such as milk whey protein, milk-derived protein like casein and soybean protein; flavonoides such as catechin and isoflavone; proteoglycans such as chondroitin sulfate and gentamycin sulfate; extracts or galenicals of such as clove, juniper berry and arborvitae. Also, combined use is possible with, for example, a thickening agent, a gelatinizing agent, carrageenan as an emulsion stabilizer, carboxymethylcellulose, guar gum, pectin, xanthan gum, arabic gum, locust bean gum, tragacanth gum, gelin gum, alginic acid, dextran, pullulan and curdlan.

Administration dose and intake of a vitamin D-like active agent and an improving agent for intestinal function, of the present invention, is 0.05 to 15 g, preferably 0.1 to 10 g for each adult per day as a sugar-phosphate ester or a salt thereof.

The present invention can provide an alternative agent with vitamin D-like activity having high safety for long-term administration. Also, using an improving agent for intestinal function, age-related depression of digestion and absorption ability of intestinal canal can be restored.

### Examples

### Example 1

As a target cell of 1α,25-dihydroxyvitamin D and a sugar-phosphate ester, Caco-2 cells (from Dainippon Pharmaceutical Co. Ltd.) were employed. Caco-2 cells are known to differentiate to epitherial-like cells of small intestine. The Caco-2 cells were suspended in culture medium (10 % fetal calf serum, none essential amino acids for MEM, 200 mmol/L glutamic acid/MEM Earle) and added to an insert of a fibrillar collagen culture plate (from Becton Dickinson Inc.). To the plate also, the culture medium was added, and cultured according to a usual method. The media in the insert and the plate were renewed at a regular interval, and the culture was subjected to a test at 15 days of incubation. Then, 10 nmol/L of 1α,25-dihydroxyvitamin D (from Sigma-Ardrich Japan Co., Ltd.) or 1 mmol/L of glucose 1-phosphate sodium salt (from Solchem Co., Italy), as a monosaccharide phosphate salt, was added to the insert, and cultured for 72 hours, while exchanging the medium by every 24 hours, followed by extraction of mRNA of Caco-2 cells using Isogen (from Wako Pure Chemical Ind. Ltd.) and assaying the amount of mRNA of the targeting genes, comprising CYP3A gene, 24-hydroxylase gene and CaT1 gene, by means of a RT-PCR method using specific primer sequence for each targeting gene.

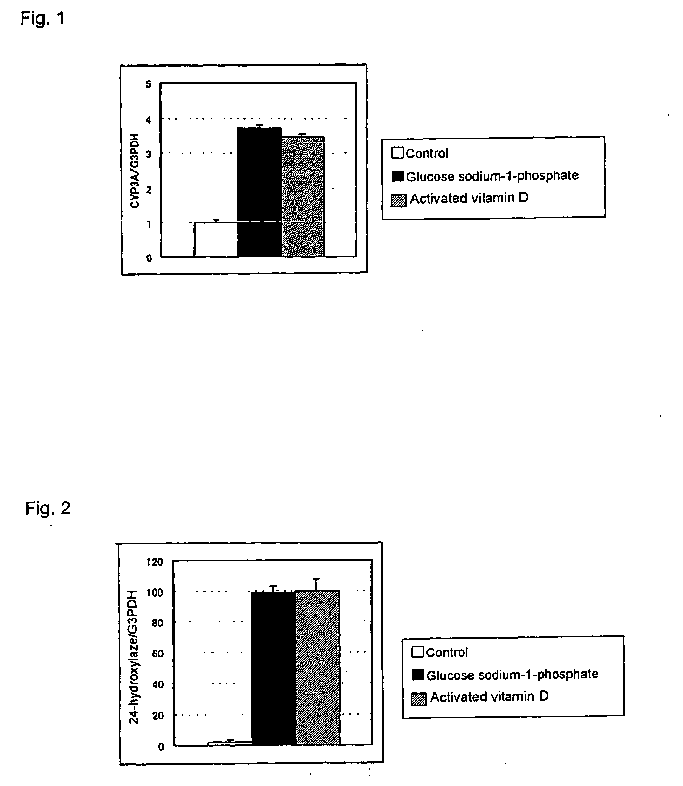

As is clear from the results shown in Figs. 1 to 3, glucose 1-phosphate sodium salt significantly enhanced gene expression of 24-hydroxylase, CYP3A and CaT1, similarly as in 1α,25-dihydroxyvitamin D, (the amount of each gene expression was divided by housekeeping gene, G3PDH). From these facts in evaluation of vitamin D-like activity using a protein molecule, whose gene expression is controlled via VDR, as an indicator, glucose 1-phosphate sodium salt, one of a monosaccharide phosphate ester, was found to have activity equivalent to that of 1α,25-dihydroxyvitamin D and can be an alternative agent with vitamin D-like activity.

### Example 2

The aged rats (9 month aged, male, SD-IGS) were divided into two groups; (1) control group, (2) glucose-1-phosphate group. Each group was fed for 10 days, and feeding stuff prescription is shown in Table 1 below. Concentrations of Ca and P were fixed to 0.5 % by weight and 0.4 % by weight, respectively, and glucose 1-phosphate sodium salt from Solchem Co. (Italy) was used as a representative a sugar-phosphate salt. On 10 days after the test started, rats were made starved by fasting for all day and night, then the small intestines were taken out, followed by extraction, immediate opening of the intestinal canal and stripping off the epitherial mucous membrane to prepare protein extract samples according to the method described by H. J. Armbrecht et al. (Am. J. Physiol., 236(6), e769-e774, 1979). These samples were separated by SDS-PAGE, followed by transferring on PVDF membrane (IPVH, pore size of 0.45, Millipore) in accordance with a usual procedure and assaying of calbindin D9K by the Western blotting method. Rabbit anti-calbindin D9K antibody (from Research Diagnostic Inc.) as the primary antibody and HRP anti-rabbit IgG (Bio-Rad) as the secondary antibody were used, and detected using ECL Plus kit (Bio-Rad). The detected protein bands were further quantified using Scion Image-analyzing software (from Scion Corp.).

**Table 1**

| Feeding stuff prescription | | |
|---|---|---|
| Composition (% by weight) | Control group | Test group |
| Casein | 20.0 | 20.0 |
| DL-Methionine | 0.3 | 0.3 |
| α-Potato starch | 15.0 | 15.0 |
| Sucrose | 47.2 | 46.0 |
| Cellulose powder | 5.0 | 5.0 |
| Corn oil | 5.0 | 5.0 |
| Minerals (Ca-free) | 3.5 | 3.5 |
| Calcium carbonate | 1.2 | 1.2 |
| Glucose 1-phosphate sodium salt | - | 1.9 |
| Potassium dihydrogenphosphate | 1.8 | 1.1 |
| Vitamins (AIN 76) | 1.0 | 1.0 |
| Total | 100 | 100 |

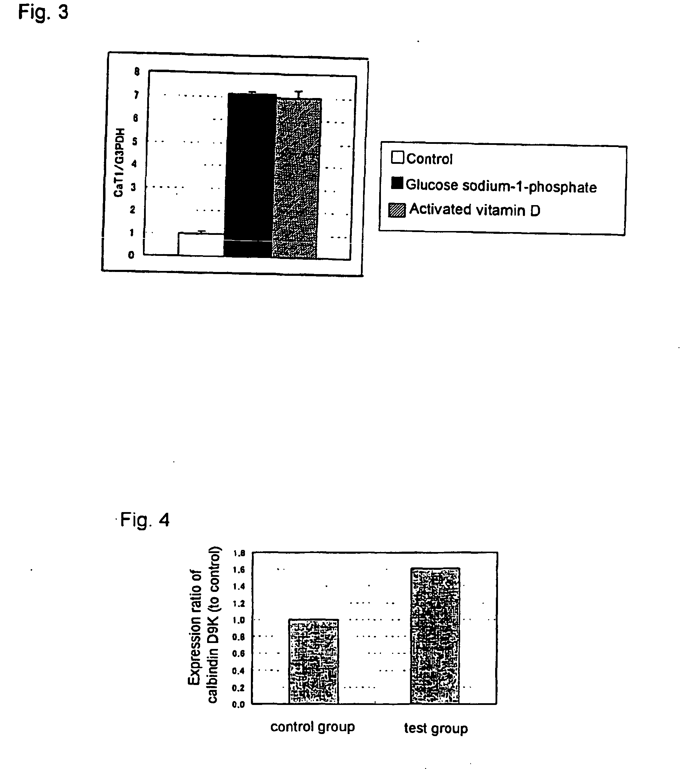

As is clear from the results shown in Fig.4, calbindin D9K expression in the test group was significantly enhanced compared to that of the control group.

As described above, in evaluation of anti-aging activity using calbindin D9K, whose gene expression in small intestine is known to be depressed by aging, as an indicator, glucose 1-phosphate sodium salt, one of a monosaccharide phosphate ester, was found to significantly enhance expression of calbindin D9K in small intestine, and have activity to recover depressed function of small intestine.

### Example 3

The following foods could be manufactured using glucose 1-phosphate sodium salt, which is an alternative agent with vitamin D-like activity and an improving agent for age-related depression of intestinal function.

### (1) Food supplement

Glucose 1-phosphate sodium salt of 60 % by weight and dextrin of 40 % by weight were mixed and packed to give each 2 g package, or 2 g tablet by formulation of the mixture in a usual manner, so as to provide convenient food supplement which can easily be used in cooking as one package or one tablet for one-person-meal.

### (2) Powder beverages

To the mixture of 60 % by weight of glucose, 15 % by weight of dextrin, 5 % by weight of sodium citrate, 1 % by weight of vitamin C and 15 % by weight of glucose 1-phosphate sodium salt, 4 % by weight of flavoring agent (lemon flavor) was added, mixed well and packed to give each 10 g package. The packaged powder was dissolved in water or hot water of predetermined volume to be taken once a day, to obtain good flavoring powder beverage.

### (3) Tablet cookie

Sixty (60) % by weight of anhydrous glucose crystal, 9 % by weight of frost sugar, 4.5 % by weight of powder orange flavoring agent, 2 % by weight of guar gum, 2.5 % by weight of ascorbic acid, 1.5 % by weight of citric acid (crystal), 5 % by weight of milk calcium, 15 % by weight of glucose 1-phosphate sodium salt, 0.5 % by weight of sucrose fatty acid ester (HLB20) and proper quantity of pigment were mixed homogeneously, and 2 g tablets with 15 mm of diameter were produced in a usual manner to obtain good favored tablet cookie which is consumable 5 tablets per day.

### (4) Jelly food

To 40 % by weight of water, 0.65 % by weight of a mixed gelatinizing agent of carrageenan and locust bean gum, 10 % by weight of 50 % concentrated grapefruit juice, 0.05 % by weight of citric acid, 0.05 % by weight of vitamin C, 1.5 % by weight of glucose 1-phosphate sodium salt were added, followed by the addition of a proper quantity of grapefruit flavor and liquid sugar, and further addition of water to make 1 kg of a 100 % by weight solution. After germicidal treatment at 85 °C for 5 minutes, aliquots of the solution were placed in 100 mL volume vessels, and gelatinized by standing with slow cooling at 5 °C. Thus, jelly food having good melting property in mouth, fruity flavor, and good feeling of taste on eating can be obtained.

### (5) Milk beverage

Thirty(30) g of glucose 1-phosphate sodium salt was dissolved in 5 kg of commercially available caw milk, and 200 mL aliquots are canned and sterilized in a usual manner to obtain milk beverage.

### (6) Refreshing drink

Thirteen (13) % by weight of liquid sugar of fructose and glucose, 0.3 % by weight of citric acid, 0.03 % by weight of ascorbic acid, 0.02 % by weight of sodium citrate, 0.1 % by weight of fragrance (lime flavor), 0.5 % by weight of calcium carbonate and 1.2 % by weight of glucose 1-phosphate sodium salt were mixed and dissolved in water, followed by adjustment of pH to 3.5, and filling up to make 5 L of beverage. Each 100 mL aliquot is placed in a glass bottle, and sterilized in a usual manner to obtain refreshing drink with good flavor and taste.

### (7) Water

Thirty (30) g of glucose 1-phosphate sodium salt was dissolved in 5 kg of commercially available mineral water. After filtration, 200 mL aliquots of the solution were filled in a can, and sterilized in a usual manner to obtain can-packed water.

### (8) Tea beverage

To 5 kg of tea beverage, prepared by extracting from 50 g of oolong tea leaves, 20 g of glucose 1-phosphate sodium salt and 1 g of sodium ascorbate were added and dissolved, adjusted pH to 5.5 using sodium bicarbonate, then a 350 ml aliquots is packed in a can and sterilized in a usual manner to obtain flavorous oolong tea.

### (9) Coffee beverage

To 4 kg of coffee, prepared by extracting from 250 g of roasted coffee beans (Moca, Colombia), 200 g of sucrose, 500 g of milk and glucose 1-phosphate sodium salt were added and dissolved, followed by adjustment of pH to 6 using sodium bicarbonate, filling-up with water to make 5 kg solution, packing of 200 ml aliquot in a can and sterilization in a usual manner to obtain good flavorous coffee beverage.

### (10) Vegetable-containing mixed fruit juice beverage

Twelve (12) g of glucose 1-phosphate sodium salt was added and dissolved in 2 kg of commercially available mixed fruit drink, followed by packing of 200 ml aliquot in a can and sterilization in a usual manner to obtain vegetable-containing mixed fruit juice beverage.

### (11) Ice cream

To 2 kg of caw milk, 60 g of sucrose, 30 g of glucose 1-phosphate sodium salt and a proper quantity of flavor were added, followed by stirring and cooling to prepare cream. The cream was divided into 20 portions and further cooled down to obtain flavorous taste ice cream.

### (12) Wheat flour foods (cupcake)

Dough, consisting of 100 g of weak flour, 100 g of chicken egg, 100 g of sugar, 35 g of shortening, 35 g of margarine, 2.5 g of baking powder, 6 g of wine or spirit, 15 g of glucose 1-phosphate sodium salt and a proper quantity of water, was prepared by the same way as for cupcake, followed by die-cutting to 10 pieces and baking in a usual manner to obtain flavorous and good taste cupcakes.

### (13) Seasoning (soy sauce)

Hundred (100) g of glucose 1-phosphate sodium salt was dissolved in 1 kg of commercially available soy sauce or low-salt soy sauce, and sterilized. Thus, soy sauce or low-salt soy sauce by which the consumption volume is controlled to be 20 g per day can be obtained.

### (14) Health drink

Eight hundreds (800) mg of taurine, 11000 mg of sucrose, 50 mg of caramel, 30 mg of sodium benzoate, 5 mg of vitamin B1 nitrate salt, 20 mg of vitamin B2, 20 mg of vitamin B6, 2000 mg of vitamin C, 100 mg vitamin E, 20 mg of nicotinic acid amide and 1200 mg of glucose 1-phosphate sodium salt were dissolved in a proper amount of purified water, followed by adjustment of pH to 3 using an aqueous solution of phosphoric acid, filling up to 100 ml with purified water and sterilization by heating at 80 °C for 30 minutes to obtain good flavorous health drink.

## Claims

1. Use of a monosaccharide phosphate ester or a salt thereof for the manufacture of an agent for providing vitamin D-like activity for preventing or curing skin disease, parathyroid disease, malignant tumor and bone disease.

2. Use of a monosaccharide phosphate ester or a salt thereof for the manufacture of an agent for the improvement of age-related depression of intestinal function.

3. Use according to claim 1 or 2, wherein the monosaccharide phosphate ester salt is a sodium salt.

4. Use according to claim 3, wherein the monosaccharide phosphate ester or a sodium salt thereof is glucose-phosphate ester or a sodium salt thereof.

5. Use according to claim 4, wherein the glucose-phosphate ester or a sodium salt thereof is glucose 1-phosphate or a sodium salt thereof.

## Patentansprüche

1. Verwendung eines Monosaccharidphosphatesters oder eines Salzes davon zur Herstellung eines Mittels zum Schaffen einer Vitamin D-artigen Aktivität zur Verhinderung oder Heilung von Hauterkrankungen, Parathyroiderkrankung, malignem Tumor und Knochenerkrankung.

2. Verwendung eines Monosaccharidphosphatesters oder eines Salzes davon zur Herstellung eines Mittels zur Verbesserung der altersbezogenen Depression der Intestinalfunktion.

3. Verwendung nach Anspruch 1 oder 2, worin das Monosaccharidphosphatestersalz ein Natriumsalz ist.

4. Verwendung nach Anspruch 3, worin der Monosaccharidphosphatester oder ein Natriumsalz davon ein Glucosephosphatester oder ein Natriumsalz davon ist.

5. Verwendung nach Anspruch 4, worin der Glucosephosphatester oder ein Natriumsalz davon Glucose-1-phosphat oder ein Natriumsalz davon ist.

## Revendications

1. Utilisation d'un phosphate de monosaccharide ou de son sel pour la fabrication d'un agent destiné à assurer une activité de type vitamine D pour prévenir ou traiter les maladies de la peau, les maladies parathyroïdiennes, les tumeurs malignes et les maladies osseuses.

2. Utilisation d'un phosphate de monosaccharide ou de son sel pour la fabrication d'un agent destiné à réduire l'affaiblissement de la fonction intestinale lié à l'âge.

3. Utilisation selon la revendication 1 ou 2, dans laquelle le sel du phosphate de monosaccharide est un sel de sodium.

4. Utilisation selon la revendication 3, dans laquelle le phosphate de monosaccharide ou son sel de sodium est le phosphate de glucose ou son sel de sodium.

5. Utilisation selon la revendication 4, dans laquelle le phosphate de glucose ou son sel de sodium est le 1-phosphate de glucose ou son sel de sodium.
